# EUROPEAN PATENT SPECIFICATION

(11) **EP 2 315 024 B1**
(45) Date of publication and mention of the grant of the patent: **18.12.2013**
(21) Application number: 09761240.2
(22) Date of filing: 26.03.2009
(51) Int. Cl.: G01N 33/49, G01N 33/543

(54) **A METHOD OF QUICKLY DETERMINING HUMAN ABO/RH/MN BLOOD TYPE AND A TEST KIT THEREOF**
VERFAHREN ZUR SCHNELLBESTIMMUNG DES MENSCHLICHEN AB0/RH/MN-BLUTTYPS UND TESTKIT DAFÜR
PROCÉDÉ DE DÉTERMINATION RAPIDE DES GROUPES SANGUINS HUMAINS ABO/RH/MN ET KIT DE DOSAGE POUR CELUI-CI

(30) Priority: 10.06.2008 CN 200810111567
(43) Date of publication of application: 27.04.2011
(73) Proprietor: Intec Products, Inc. (Xiamen), Fujian 361022 (CN)
(72) Inventor: WANG, Daming, Xiamen Fujian 361022 (CN); YANG, Wen, Xiamen Fujian 361022 (CN); XIAO, Jiangqun, Xiamen Fujian 361022 (CN); WANG, Baodan, Xiamen City, Fujian Province 361022, P.R. (CN)
(74) Representative: Eisenführ, Speiser & Partner
(86) International application number: PCT/CN2009/000322
(87) International publication number: WO 2009/149615

(56) References cited:
- EP-A1- 0 223 978
- CN-A- 1 438 486
- GB-A- 2 250 342
- US-A1- 2007 248 983
- US-A1- 2008 085 525
- FREDV. PLAPP ET AL: "DIPSTICKS FOR DETERMINING ABO BLOOD GROUPS", THE LANCET, vol. 327, no. 8496, 28 June 1986 (1986-06-28), pages 1465-1466, XP55000045, ISSN: 0140-6736, DOI: 10.1016/S0140-6736(86)91501-1
- YU WEI: 'A test-strip method for determining blood type' FOREIGN MEDICAL INFORMATION no. 1, 1987, page 17, XP008141924

## Description

### Technical Field

The present invention relates to a method for rapidly detecting human ABO/Rh/MN blood group and a kit thereof as defined in the claims.

### Background of the Invention

Blood typing is a conventional technique for grouping human red blood cells according to ABO system with four different groups of A, B, AB and O, according to Rh system and MN blood group system respectively. A general procedure for identifying blood group is to mix red cells to be detected with a reagent containing a blood group antibody, and, after the reaction of the above, to determine the blood group of the red cells detected by observing the occurrence of red cell agglutination. Such a procedure comprises firstly isolating red cells from a patient's serum, then mixing an appropriate amount of the red cells with respective test solutions introduced into a test tube or slide (containing anti-A, anti-B, anti-D, anti-M or anti-N serum respectively), and optionally adding further regents promoting immune reaction and incubating the reaction, then centrifuging and agitating the reaction mixture followed by observing the occurrence of red cell agglutination.

For a sample, if the agglutination occurs in the presence of an anti-A reagent, while no agglutination occurs in the presence of an anti-B reagent, then the blood sample is identified as a blood group of A. If red cell agglutination occurs in the presence of an anti-B reagent, while no agglutination occurs in the presence of an anti-A reagent, then the sample is identified as a blood group of B. If red cell agglutination occurs both in the presence of an anti-A reagent and in the presence of an anti-B reagent, then the sample is identified as a blood group of AB. If neither agglutination reaction occurs in the presence of an anti-A reagent or in the presence of an anti-B reagent, then the sample is identified as a blood group of O. If the agglutination occurs in the presence of an anti-D reagent, then the sample is Rh-positive blood group, otherwise it is Rh-negative blood group. For MN blood group system, a sample can be designated to be of M, N or MN blood group according to the occurrence of red cell agglutination.

Current technologies used to determine human ABO/Rh/MN blood group can be classified into two types. The first type is based on the detection of blood group antigen associated with cell membrane, and includes red cell saline agglutination assay, neutralization assay, absorption assay, dissociation assay and mixed agglutination reaction. The second type is based on direct detection of free blood group antigen, and includes coaglutination reaction and radioimmunoassay.

Currently in commonly used red cell saline agglutination assay, the test blood is determined with its blood group by red cell agglutination induced by the binding of known IgM or IgG antibody to blood group antigen on the surface of fresh red cells. This procedure is widely used in blood group determination (blood typing) for clinical blood transfusion. However, this procedure needs corresponding reagents, which should be preserved at a low temperature, and furthermore, its result is difficult to be confirmed.

Other procedures for determining blood group have various shortcomings. For example, neutralization assay is carried out in a period of long time and has a poor sensitivity. Dissociation test and mixed agglutination reaction require highly skilled operation with complicated optimization in each run and have a poor sensitivity. Coagglutination reaction comprises sensitizing protein A from *Staphyloccocus aureus* (SPA) by IgG antibody, then observing the agglutination of *Staphyloccocus aureus* to determine the blood group of the sample tested, while this procedure requires complicated storage of reagents. Radioimmunoassay and ELISA are highly sensitive, while have a long time to run, and thereby are not suitable for a single sample or for on-site detection.

Judith Parke and Deborah Lynne Morris (GB2250342A, 1992) disclose a method and device for typing human blood groups based on red blood cell agglutination. Hydrophilic PE is used in the device as the material for blood sample application. In addition, multiple application zones were arranged along the test strip.

D.E. Nevalainen et al (EP0223978, 1987) dislcose methods and devices for determining blood group classifications from blood or serum samples. A porous solid substrate having an average pore size of 0.025 to 7 µm is used in the devices as that for blood loading.

Peter Schwind and Klemens Loster (US2007/0248983A1, 2007) disclose a device and method for simultaneously identifying blood group antigens. The device has an application zone and an indicator zone region in two separate locations. A porous organic material such nitrocellulose, polyethylene or nylon is used as the material for blood application.
In summary, commonly used methods for clinical blood group determination has a variety of problems such as long operation time, labor-intensive work, a subjective result with a great variation, and potential contamination. Therefore, it is necessary to make a substantive improvement of conventional methods for blood group determination.

### Summary of the Invention

An object of the present invention is to provide a kit for carrying out a method for rapidly detecting human ABO/Rh/ MN blood group, the kit consisting of a test strip, a rinse solution and a blood-collecting means, wherein the test strip consists of a rinse pad, a loading pad, a cushion pad, an absorptive pad, a water-proof adhesive tape and a polyester sheet as a backing, and wherein the rinse pad, the loading pad, the cushion pad and the absorptive pad which form the test strip are made of a porous polymeric fibre glass material having an appropriate pore size for at least one red cell passing through,
wherein the rinse pad, the loading pad, the cushion pad, the absorptive pad and the water-proof adhesive tape are sequentially attached to the backing, the edge of the loading pad overlaps on the edge of the rinse pad, the edge of the cushion pad overlaps on the edge of the loading pad, the edge of the absorptive pad overlaps on the edge of the cushion pad, and the water-proof adhesive tape is applied onto the overlapped edges of the rinse pad and the loading pad, so as to prevent the rinse solution from overflowing from the loading pad,
the method comprising the steps of dripping a blood sample onto the loading pad of the test strip pre-coated by immobilization with anti-A or anti-B or anti-D or anti-M or anti-N antibody, dripping a rinse solution onto one end of the test strip so as to carry unagglutinated red cells along with the rinse solution away from the loading site, while leave the red cells agglutinated with the antibody remained at the loading site; then determining the blood group of the sample according to the presence of agglutinated red cells at the loading site as reaction site.

Another object of the invention is to provide a method for rapidly detecting human ABO/Rh/MN blood group using the kit of the invention, comprising the steps of dripping a blood sample onto the loading pad of the test strip pre-coated by immobilization with anti-A or anti-B or anti-D or anti-M or anti-N antibody, dripping a rinse solution onto one end of the test strip so as to carry unagglutinated red cells along with the rinse solution away from the loading site, while leave the red cells agglutinated with the antibody remained at the loading site; then determining the blood group of the sample according to the presence of agglutinated red cells at the loading site as reaction site.

According to a preferred embodiment of the invention, the blood sample dripped on the test strip may have a volume as small as 1-10 µl. Said rinse solution is 10 mM phosphate buffer saline (PBS), physiological saline or other isotonic solution. Said blood sample to be tested is dripped on a loading pad of the test strip.

According to a preferred embodiment of the invention, the method for immobilizing the blood group antibody may be, for example, based on physical adsorption or chemical crosslinking.

According to a preferred embodiment of the invention, said blood sample to be tested may be red cells or whole blood, or a suspension of red cells or whole blood diluted with an isotonic solution such as physiological saline or phosphate buffer saline. In addition, said blood sample includes a partially hemolyzed blood sample and a blood sample rich in lipid.

According to a preferred embodiment of the invention, the test results can be determined visually or by virtue of a device for collecting photo or electric signal.

Briefly, a blood sample contacts and reacts with the loading pad immobilized with a corresponding blood group antibody, i.e., the blood sample undergoes an immunological binding reaction with the corresponding antibody on the loading pad. After rinsed with a rinse solution, the blood sample which has undergone immunological binding reaction remains on the pad and has a significant red color, while the blood sample which has undergone no immunological binding reaction is rinsed away from the loading pad by the rinse solution and thereby no red color appears at the loading site. Therefore, the ABO/Rh/MN blood group of a blood sample can be determined by the occurrence of the immunological reaction between red cells in the sample and the blood group antibody, which is determined by the occurrence of red substance on the loading pad after the immune reaction between red cells and the blood group antibody.

### Brief Description of the Drawings

Figure 1 depicts an illustrative fast detection strip for ABO blood group in the blood group detection kit of the present invention.
Figure 2 depicts a disassembly view of a further illustrative fast detection strip for ABO blood group in the blood group detection kit of the present invention, which comprises a rinse pad 1, a water-proof adhesive tape 2, a loading pad 3 fixed with blood group antibody, a cushion pad 4, an absorptive pad 5, and a polyester backing sheet 6.
Figure 3 depicts an illustrative fast detection strip for Rh blood group in the kit of the present invention, which comprises a rinse pad 1, a water-proof adhesive tape 2, a loading pad 3 fixed with anti-D antibody, a cushion pad 4, and an absorptive pad 5.
Figure 4 depicts an illustrative fast detection strip for MN blood group in the blood group detection kit of the present invention.

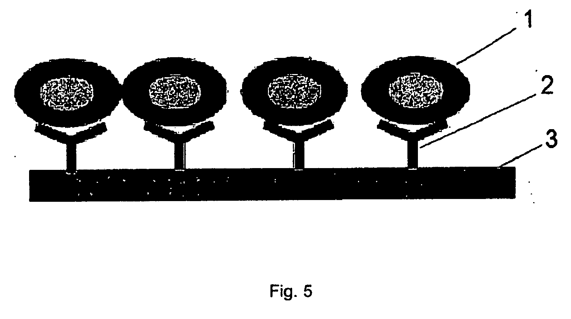

Figure5 depicts a schematic reaction after dripping positive blood sample on a fibre glass pad fixed with a blood group antibody, in which 1 indicates red cells, 2 indicates blood group antibody, and 3 indicates a fibre glass loading pad.

### Detailed Description of the Invention

The present invention pertains to a method for rapid detecting ABO/Rh/MN blood group of a human blood sample to be tested and a kit thereof as defined in the claims.

An object of the present invention is to provide a kit for carrying out a method for rapidly detecting human ABO/Rh/ MN blood group, the kit consisting of a test strip, a rinse solution and a blood-collecting means, wherein the test strip consists of a rinse pad, a loading pad, a cushion pad, an absorptive pad, a water-proof adhesive tape and a polyester sheet as a backing, and wherein the rinse pad, the loading pad, the cushion pad and the absorptive pad which form the test strip are made of a porous polymeric fibre glass material having an appropriate pore size for at least one red cell passing through,
wherein the rinse pad, the loading pad, the cushion pad, the absorptive pad and the water-proof adhesive tape are sequentially attached to the backing, the edge of the loading pad overlaps on the edge of the rinse pad, the edge of the cushion pad overlaps on the edge of the loading pad, the edge of the absorptive pad overlaps on the edge of the cushion pad, and the water-proof adhesive tape is applied onto the overlapped edges of the rinse pad and the loading pad, so as to prevent the rinse solution from overflowing from the loading pad,
the method comprising the steps of dripping a blood sample onto the loading pad of the test strip pre-coated by immobilization with anti-A or anti-B or anti-D or anti-M or anti-N antibody, dripping a rinse solution onto one end of the test strip so as to carry unagglutinated red cells along with the rinse solution away from the loading site, while leave the red cells agglutinated with the antibody remained at the loading site; then determining the blood group of the sample according to the presence of agglutinated red cells at the loading site as reaction site.

Another object of the invention is to provide a method for rapidly detecting human ABO/Rh/MN blood group using the kit of the invention, comprising the steps of dripping a blood sample onto the loading pad of the test strip pre-coated by immobilization with anti-A or anti-B or anti-D or anti-M or anti-N antibody, dripping a rinse solution onto one end of the test strip so as to carry unagglutinated red cells along with the rinse solution away from the loading site, while leave the red cells agglutinated with the antibody remained at the loading site; then determining the blood group of the sample according to the presence of agglutinated red cells at the loading site as reaction site.

According to a preferred embodiment of the invention, said blood sample to be tested may be red cells or whole blood, or a suspension of red cells or whole blood diluted with an isotonic solution such as physiological saline or phosphate buffer saline (PBS). In addition, said blood sample may include a partially hemolyzed blood sample and a blood sample rich in lipid.

According to a preferred embodiment of the invention, the volume of the blood sample dripped on the test strip is not more than 10 µl. Said rinse solution is 10 mM phosphate buffer saline, physiological saline or others.

According to a preferred embodiment of the invention, the method for fixing the blood group antibody may be, for example, based on physical adsorption or chemical cross-linking.

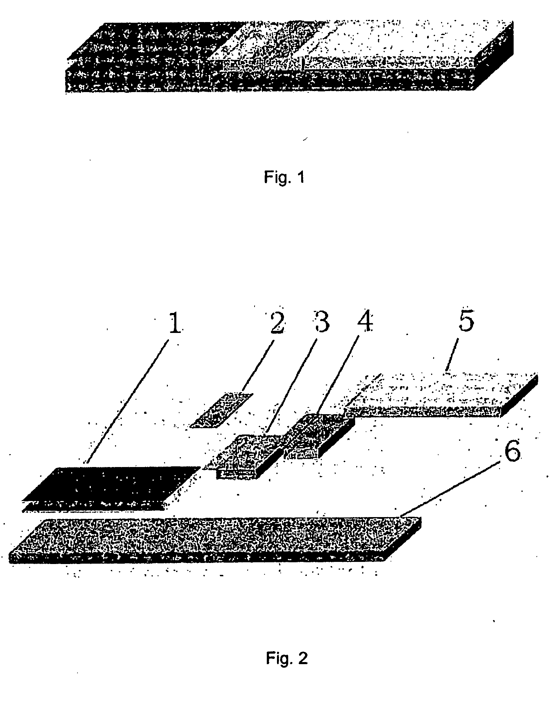

A test-strip for use in the invention illustrated on figure 1.

In the invention, said rinse pad, loading pad, cushion pad and absorptive pad are made of porous polymeric fibre glass materials in which the pore size allows at least a single red cell passing through. In view of the selected material, the rinse pad, the loading pad and the cushion pad sometimes are generally referred as fibre glass pad.

According to a preferred embodiment of the invention, the blood sample to be tested is dripped onto the loading pad, and said rinse solution is dripped onto the rinse pad. The loading pad is a porous support fixed with a blood group antibody. In addition, the method for fixing the blood group antibody may be based on physical adsorption or chemical cross-linking.

According to a preferred embodiment of the invention, said blood sample may be a partially hemolyzed blood sample or a blood sample rich in blood lipid.

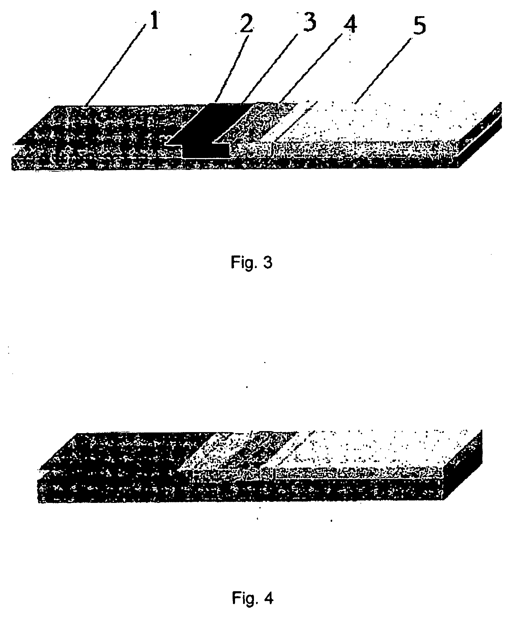

According to a preferred embodiment of the invention, the test results can be determined visually or by virtue of a device for collecting and reading photo or electric signal. Specifically, using the kit of the present invention, the procedure for detecting human ABO/Rh/MN blood group comprises:
1) Directly or indirectly fixing human ABO/Rh/MN blood group antibody on the loading pad 3 (Fig. 2): fixing the blood group antibody on fibre glass pad with a pore size by using physical adsorption or chemical crosslinking. The selected physical or chemical fixing method has little effect on biological activity of the antibodies. Furthermore, the fixed antibody is supplemented with a protein protective agent, which may be selected from the group consisting of bovine serum albumin, calf serum, peptone, sucrose, polyvinylpyrrolidone, and the combination thereof.
   Diluting human ABO/Rh/MN blood group monoclonal antibody of high titer with 5 mM phosphate buffer saline to a proper titer, adding a antibody protective agent to facilitate the fixation and preservation of the antibody on the fibre glass pad. A reaction enhancer may be added into the diluted antibody solution to promote subsequent immune reaction. Applying the diluted antibody on the pre-activated fibre glass pad in a ratio of 1.5 ml antibody per 5cm×5cm fibre pad and then preserving in seal after drying at 37°C.
2) Dripping the test sample onto the loading pad 3, followed by dripping the rinse solution onto the rinse pad 2 (see Fig. 3). After loading the sample and dripping the rinse solution, an immune binding reaction occurs and the result is presented.
3) After completing the reaction and a further rinse, determining the blood group of the test sample according to the presence of red residue on the loading pad.

For an ABO blood group test strip, after the reaction is rinsed, if a substantial amount of red substance remains on the loading pad containing anti-A antibody, it indicates that the blood sample is captured by the anti-A antibody and thereby agglutination reaction therebetween occurs (i.e., positive reaction); if no substantial amount of red substance remains on the loading pad containing anti-A antibody, it indicates that no agglutination reaction occurs between the blood sample and the anti-A antibody (i.e., negative reaction); if a substantial amount of red substance remains on the loading pad containing anti-B antibody upon rinsing the completed reaction, it indicates that an agglutination reaction occurs between the blood sample and the anti-B antibody; if no substantial amount of red substance remains on the loading pad containing anti-B antibody upon rinsing the completed reaction, it indicates that no agglutination reaction occurs between the blood sample and the anti-B antibody. Therefore, it can be determined that, according to the test results, a blood sample which produces agglutination reaction with both anti-A and anti-B antibodies is of AB blood group; a blood sample which produces agglutination reaction merely with anti-A antibody is of A blood group; if a blood sample which produces agglutination reaction merely with anti-B antibody is of B blood group; and a blood sample which produces no agglutination reaction with anti-A or anti-B antibody is of O blood group.

For an Rh blood group test strip, after the reaction is rinsed, if a substantial amount of red substance remains on the loading pad containing anti-D antibody, it indicates that agglutination reaction occurs between the blood sample and the anti-D antibody; while if no substantial amount of red substance remains on the loading pad containing anti-D antibody, it indicates that no agglutination reaction occurs between the blood sample and the anti-D antibody. Therefore, it can be determined that, according to the test results, a blood sample which produces agglutination reaction with anti-D antibody is of Rh-positive blood group, while a blood sample which produces no agglutination reaction with anti-D antibody is of Rh-negative blood group.

For an MN blood group test strip, after the reaction is rinsed, if a substantial amount of red substance remains on the loading pad containing anti-M antibody, it indicates that the blood sample is captured by the anti-M antibody, and thereby agglutination reaction occurs therebetween; if no substantial amount of red substance remains on the loading pad containing anti-M antibody, it indicates that no agglutination reaction occurs between the blood sample and the anti-M antibody ; if a substantial amount of red substance remains on the loading pad containing anti-N antibody, it indicates that agglutination reaction occurs between the blood sample and the anti-N antibody. Therefore, it can be determined that, according to the test results, a blood sample which produces agglutination reaction with both anti-M and anti-N antibody is of MN blood group; a blood sample which produces agglutination reaction merely with anti-M antibody is of M blood group; and a blood sample which produces agglutination reaction merely with anti-N antibody is of N blood group.

In the present invention, said fast test strip consists of a backing, a rinse pad, a loading pad, a cushion pad, an absorptive pad and a water-proof adhesive tape. The rinse pad, the loading pad, the cushion pad, the absorptive pad and the water-proof adhesive tape are sequentially attached to the backing. The edge of the loading pad overlaps on the edge of the rinse pad, the edge of the cushion pad overlaps on the edge of the loading pad, the edge of the absorptive pad overlaps on the edge of the cushion pad, while the water-proof adhesive tape is applied onto the overlapped edges of the rinse pad and the loading pad so as to prevent the rinse solution from overflowing from the loading pad. The backing is made of a polyester sheet.

According to a preferred embodiment of the present invention, the term "fixing an antibody"is equivalent to "immobilizing an antibody" and means fixing (immobilizing) anti-A or anti-B or anti-D or anti-M or anti-N antibody onto a support such as a fibre glass pad having a pore size or other support having a similar pore size, for example by means of physical or chemical crosslinking. Prior to the fixation, the antibody may be diluted in a ratio in light of antibody titer, so as to achieve a proper concentration that can be used. The antibody may be fixed onto the fibre glass pad (loading pad) for example by soaking or applying uniformly or spraying, then be subjected to freeze drying or other drying. Said antibody may be pretreated with glutaraldehyde as crosslinking agent.

According to a preferred embodiment of the present invention, said blood sample to be tested may be red cells or whole blood, or a suspension of red cells diluted with a isotonic solution such as 10 mM phosphate buffer saline (PBS) and physiological saline (usually diluted to 2-50%). A blood sample partially hemolyzed or rich in blood lipid may also be applicable for the kit of the present invention.

A test sample of whole blood or blood cells either diluted or undiluted may be added to the loading pad fixed with a blood group antibody, after allowing immunological binding reaction, the loading pad is rinsed with a rinse solution. Said rinse solution may be 10 mM phosphate buffer saline (PBS) or other. After allowing immune reaction, the presence of agglutinated red cells can be readily observed visually, or the test results of samples in batch can be read out by a device for collecting and reading optical or electric signal.

For the detection of ABO blood group, operations of the present method can be practiced on two test strips which are separately and in parallel formed on the same polyester backing sheet and are fixed on the loading pad respectively with anti-A or anti-B blood group antibody.

For the detection of MN blood group, operations of the present method can be practiced on two test strips which are separately and in parallel formed on the same polyester backing sheet and fixed on the loading pad respectively with anti-M or anti-N blood group antibody.

The fast detection kit for ABO/Rh/MN blood group of the present invention is suitable for blood group screening at a blood bank, field blood collection and household self-test for ABO/Rh/MN blood group. Rapid and credible blood group identification can be achieved by using rapid immune reaction and simple result reading-out. The present method is easy to perform and no additional reagent such as anti-human globulin reagent is needed. In the present method, the loading volume of the blood sample may be not more than 10 µl, and the whole duration may be not more than 2 minutes.

The method for fast detecting human ABO/Rh/MN blood group of the present invention and the kit thereof are based on the principle of immunochromatography, in which blood group determination is made by the presence of red substance due to red cell agglutination upon blood group substance antigen-antibody reaction. The present method is easy to perform and avoids various defects from the existing common methods for blood group detection in the art. The present method is suitable either for single sample detection or for multiple samples detection, especially for ABO/Rh/MN blood group detection at blood collection site and household self-detection of blood group. In combination with a relevant device, the present method can be used for rapid population screening of ABO/Rh/MN blood group in batch at a blood bank.

### Examples

The following examples are intended to further illustrate the present invention, and these examples will not limit the present invention in any way.

### Example 1: The preparation of test strips of the kit for human ABO blood group detection and the detection steps thereof

### (1) Fixing blood group antibody (anti-A, anti-B antibody) onto the loading pad:

Monoclonal anti-A and anti-B antibodies of high titer (supplied by Changchun Brother Biotech Co., Ltd) were respectively diluted with 5mM phosphate buffer saline to 1 ml with a final titer of 64. A antibody fixation protective agent consisting of 5% bovine serum albumin, 5% sucrose, 5% PVP and 5% calf serum in PBS was added to the diluted antibody solution, so as to promote the fixation and preservation of the antibody on the fibre glass pad. The diluted antibody was spreaded onto a pre-activated fibre glass pad in a ratio of 1.5 ml of the diluted antibody per 5cm × 5cm of the fibre glass pad, and the antibody was then dried at 37 °C and preserved in seal.

### (2) Assembling the fast test strips:

A rinse fibre glass pad with the width of 20 mm was attached onto the center of a polyester backing sheet with the width of about 5-10 cm, then a loading pad with the width of 10mm, a cushion pad and an absorptive pad with the width of 30mm were sequentially attached on both sides, and finally the overlapped portion of the rinse pad and the loading pad was adhesively fixed with a water-proof adhesive tape, so as to prevent the attached pads from peeling out and to prevent the rinse solution from spreading. Then the polyester sheet attached with the said pads was cut into strips with the width of 5 mm, and thereby test strips with the length of about 5-10 cm and the width of about 5 mm were obtained (see Figure1 and Figure 2).

### (3) Detecting the sample:

5 µl of a whole blood sample was dripped onto the loading pad of the test strip. After the dripping of the test sample, 5 drops of a rinse solution was slowly and in a uniform speed dripped onto the rinse pad 2. The color of the loading pad was observed after the chromatography of the rinse solution. That a substantial amount of red substance remains on the loading pad 3 indicates a positive reaction; while that no substantial amount of red substance remains on the loading pad 3 indicates a negative reaction. In combination with the knowledge about the relationship of ABO blood group antibody and red cell reaction, it is easy to determine the blood group of the test sample. Alternatively, the red residue on the loading pad can be read out by a photo signal or electric signal colleting system, then the results can be determined automatically, so as to rapidly screen the ABO blood group of blood samples in batch.

### Example 2: The preparation of test strips of the kit for human Rh blood group detection and the detection steps thereof

### (1) Fixing blood group antibody (anti-D antibody) onto the loading pad:

A monoclonal anti-D antibody of high titer (supplied by MILLIPORE Corp.) was diluted with 5mM phosphate buffer saline to 1 ml with a final titer of 32. An antibody fixation protective agent consisting of 5% albumin, 5% sucrose, 5% PVP and 5% calf serum in PBS was added to the diluted antibody solution, so as to promote the fixation and preservation of the antibody on the fibre glass pad. A reaction enhancer (2% Tween 20 and 2% Triton X-100 in PBS) was further added to promote subsequent immune reaction. The diluted antibody was spreaded onto the pre-activated fibre glass pad in a ratio of 1.5 ml of the diluted antibody per 5cm × 5cm of the fibre pad, and the antibody was then dried at 37 °C and preserved in seal (see Figure 3).

Other steps were similar to those in the preparation of test strips of the kit for human ABO blood group detection and the detection steps thereof.

### Example 3: The preparation of test strips of the kit for human MN blood group detection and the detection steps thereof.

The preparation of test strips of the detection kit for human MN blood group and the detection steps thereof are the same as those in the preparation of test strips of the detection kit for human Rh blood group and the detection steps thereof, except that the fixed antibody onto the test strip were replaced with anti-M and anti-N antibody (see Figure 4).

### Example 4: The comparison of the present method for blood group detection with the existing conventional methods.

We detected blood group on 1500 individuals from general population (blood donators) in double blind, using conventional methods for blood group detection in parallel together with and the present methods and the kit thereof. The results showed that the detection results of the present method were in consistence by 100% with those of the known method based on red cell in-suspension agglutination. Moreover, the present method can be applied to the samples in a broader range, such as partially hemolyzed blood sample and blood sample rich in lipid. Furthermore, the present method avoids the defects from the existing common method for clinical blood group identification, such as long operation time, labor-intensive work, subjective result with a great variation due to the operator, and potential contamination.

## Claims

1. A kit for carrying out a method for rapidly detecting human ABO/Rh/MN blood group,
the kit consisting of a test strip, a rinse solution and a blood-collecting means, wherein the test strip consists of a rinse pad, a loading pad, a cushion pad, an absorptive pad, a water-proof adhesive tape and a polyester sheet as a backing, and wherein the rinse pad, the loading pad, the cushion pad and the absorptive pad which form the test strip are made of a porous polymeric fibre glass material having an appropriate pore size for at least one red cell passing through,
wherein the rinse pad, the loading pad, the cushion pad, the absorptive pad and the water-proof adhesive tape are sequentially attached to the backing, the edge of the loading pad overlaps on the edge of the rinse pad, the edge of the cushion pad overlaps on the edge of the loading pad, the edge of the absorptive pad overlaps on the edge of the cushion pad, and the water-proof adhesive tape is applied onto the overlapped edges of the rinse pad and the loading pad, so as to prevent the rinse solution from overflowing from the loading pad,
the method comprising the steps of dripping a blood sample onto the loading pad of the test strip pre-coated by immobilization with anti-A or anti-B or anti-D or anti-M or anti-N antibody, dripping a rinse solution onto one end of the test strip so as to carry unagglutinated red cells along with the rinse solution away from the loading site, while leave the red cells agglutinated with the antibody remained at the loading site; then determining the blood group of the sample according to the presence of agglutinated red cells at the loading site as reaction site.

2. The kit according to claim 1, wherein the test strip is configured to allow a blood sample to be dripped onto the loading pad fixed with a blood group antibody, and an isotonic rinse solution to be dripped onto the rinse pad, then the presence of agglutinated red cells at the loading pad to be observed so as to determine the blood group of the blood sample.

3. A method for rapidly detecting human ABO/Rh/MN blood group using the kit according to claim 1, comprising the steps of dripping a blood sample onto the loading pad of the test strip pre-coated by immobilization with anti-A or anti-B or anti-D or anti-M or anti-N antibody, dripping a rinse solution onto one end of the test strip so as to carry unagglutinated red cells along with the rinse solution away from the loading site, while leave the red cells agglutinated with the antibody remained at the loading site; then determining the blood group of the sample according to the presence of agglutinated red cells at the loading site as reaction site.

4. The method according to claim 3, wherein the blood sample is selected from the group consisting of red cells, whole blood, red cells diluted with physiological saline or isotonic phosphate buffer saline and whole blood diluted with physiological saline or isotonic phosphate buffer saline.

5. The method according to claim 3, wherein the blood sample is selected from the group consisting of a blood sample that is partially hemolyzed and a blood sample that is rich in lipid.

6. The method according to claim 3, wherein the antibody is fixed onto the test strip by physical adsorption or chemical cross-linking.

7. The method according to claim 3, wherein the presence of the agglutination at the loading site as reaction site may be determined visually or by using a device collecting optical signal or electric signal.

## Patentansprüche

1. Ein Kit zum Durchführen einer Methode zum schnellen Bestimmen einer humanen ABO/Rh/MN Blutgruppe,
wobei das Kit aus einem Teststreifen, einer Spüllösung und Mittel zum Sammeln von Blut besteht,
worin der Teststreifen aus einem Spülkissen, einem Beladungskissen, einem Pufferkissen, einem absorptiven Kissen, einem wasserfesten adhäsiven Streifen und einem Polyesterblatt als Rückenstärkung besteht, und worin das Spülkissen, das Beladungskissen, das Pufferkissen, das absorptive Kissen, welche den Teststreifen bilden, aus einem porösen Fiberglasmaterial gefertigt sind, welches eine geeignete Porengröße aufweist, um zumindest eine rote Zelle hindurchpassieren zu lassen,
worin das Spülkissen, das Beladungskissen, das Pufferkissen, das absorptive Kissen und der wasserfeste adhäsive Streifen sequenziell an der Rückenstärkung befestigt sind, der Rand des Beladungskissens auf dem Rand des Spülkissens überragt, der Rand des Pufferkissens auf dem Rand des Beladungskissens überragt, der Rand des absorptiven Kissens auf dem Rand des Pufferkissens überragt, und der wasserfeste adhäsive Streifen auf die überragenden Ränder des Spülkissens und des Beladungskissens aufgebracht ist, um so die Spüllösung vor dem Überfließen vom Beladungskissen zu bewahren,
wobei die Methode die Schritte des Auftropfens einer Blutprobe auf das Beladungskissen des Teststreifens umfasst, der durch Immobilisierung mit einem anti-A oder anti-B oder anti-D oder anti-M oder anti-N Antikörper vorbeschichtet ist, des Auftropfens einer Spüllösung auf ein Ende des Teststreifens, um so nicht-agglutinierte rote Zellen zusammen mit der Spüllösung von der Beladungsseite fortzutragen, während die mit dem Antikörper agglutinierten roten Zellen an der Beladungsseite zurückgelassen bleiben; sodann das Bestimmen der Blutgruppe der Probe entsprechend der Anwesenheit von agglutinierten roten Zellen an der Beladungsseite als Reaktionsort.

2. Das Kit nach Anspruch 1, worin der Teststreifen so konfiguriert ist, um zu erlauben, eine Blutprobe auf das mit einem Blutgruppenantikörper fixierte Beladungskissen aufzutropfen, und eine isotonische Spüllösung auf das Spülkissen aufzutropfen, sodann die Anwesenheit von agglutinierten roten Zellen am Beladungskissen zu beobachten und so die Blutgruppe der Blutprobe zu bestimmen.

3. Eine Methode zum schnellen Bestimmen von humanen ABO/Rh/MN Blutgruppen unter Verwendung des Kits nach Anspruch 1, umfassend die Schritte des Auftropfens einer Blutprobe auf das Beladungskissen des Teststreifens, der durch Immobilisierung mit einem anti-A oder anti-B oder anti-D oder anti-M oder anti-N Antikörper vorbeschichtet ist, des Auftropfens einer Spüllösung auf ein Ende des Teststreifens, um so nicht-agglutinierte rote Zellen zusammen mit der Spüllösung von der Beladungsseite fortzutragen, während die mit dem Antikörper agglutinierten roten Zellen an der Beladungsseite zurückgelassen bleiben; sodann das Bestimmen der Blutgruppe der Probe entsprechend der Anwesenheit von agglutinierten roten Zellen an der Beladungsseite als Reaktionsort.

4. Die Methode nach Anspruch 3, worin die Blutprobe ausgewählt ist aus der Gruppe, bestehend aus roten Zellen, Vollblut, mit physiologischer Salzlösung oder isotonischer Phosphatpuffer-Salzlösung verdünnten roten Zellen und mit physiologischer Salzlösung oder isotonischer Phosphatpuffer-Salzlösung verdünntem Vollblut.

5. Die Methode nach Anspruch 3, worin die Blutprobe ausgewählt ist aus der Gruppe, bestehend aus einer Blutprobe, die teilweise hämolysiert ist, und einer Blutprobe, die reich an Lipiden ist.

6. Die Methode nach Anspruch 3, worin der Antikörper auf dem Teststreifen durch physikalische Adsorption oder chemische Quervernetzung fixiert ist.

7. Die Methode nach Anspruch 3, worin die Anwesenheit der Agglutinierung am Beladungsort als Reaktionsort visuell oder unter Verwendung einer Vorrichtung, die optische oder elektrische Signale aufnimmt, bestimmt werden kann.

## Revendications

1. Kit pour réaliser un procédé de détection rapide du groupe sanguin humain ABO/Rh/MN,
le kit comprenant une bande de test, une solution de rinçage et un moyen de collecte du sang, dans lequel la bande de test consiste en un tampon à rincer, un tampon de charge, un coussinet, un tampon absorbant, un ruban adhésif étanche à l'eau et une feuille de polyester comme revêtement, et dans lequel le tampon à rincer, le tampon de charge, le coussinet et le tampon absorbant qui forment la bande de test sont en un matériau poreux polymérique à fibres de verre ayant une taille de pore permettant le passage d'au moins un globule rouge,
dans lequel le tampon à rincer, le tampon de charge, le coussinet, le tampon absorbant et le ruban adhésif étanche à l'eau sont fixés l'un à la suite de l'autre sur le support, le bord du tampon de charge recouvrant le bord du tampon à rincer, le bord du coussinet recouvrant le bord du tampon de charge, le bord du tampon absorbant recouvrant le bord du coussinet et la bande adhésive résistant à l'eau étant appliquée sur les bords recouverts du tampon à rincer et du tampon de charge de façon à empêcher la solution de rinçage de s'écouler du tampon de charge,
le procédé comprenant les étapes d'écoulement d'un échantillon de sang sur le tampon de charge de la bande de test pré-revêtue par immobilisation avec un anticorps anti-A ou anti-B ou anti-D ou anti-M ou anti-N, l'écoulement d'une solution de rinçage sur une extrémité de la bande de test de façon à emporter les globules rouges non agglutinés avec la solution de rinçage hors du site de chargement, tout en laissant les globules rouges agglutinés avec l' anticorps sur le site de chargement ; puis la détermination du groupe sanguin de l'échantillon selon la présence de globules rouges agglutinés sur le site de chargement en tant que site réactionnel.

2. Kit selon la revendication 1, dans lequel la bande de test est configurée de façon à permettre à un échantillon de sang de s'écouler sur le tampon de charge fixé avec un anticorps de groupe sanguin, et à une solution de rinçage isotonique de s'écouler sur le tampon à rincer, puis de façon à observer la présence de globules rouges agglutinés sur le tampon de charge pour déterminer le groupe sanguin de l'échantillon de sang.

3. Procédé de détection rapide d'un groupe sanguin humain ABO/Rh/MN en utilisant le kit selon la revendication 1, comprenant les étapes d'écoulement d'un échantillon de sang sur le tampon de charge de la bande de test pré-revêtue par immobilisation avec un anticorps anti-A ou anti-B ou anti-D ou anti-M ou anti-N, l'écoulement d'une solution de rinçage sur une extrémité de la bande de test de façon à emporter les globules rouges non agglutinés avec la solution de rinçage hors du site de chargement, tout en laissant les globules rouges agglutinés avec l' anticorps sur le site de chargement ; puis la détermination du groupe sanguin de l'échantillon selon la présence de globules rouges agglutinés sur le site de chargement en tant que site réactionnel.

4. Procédé selon la revendication 3, dans lequel l'échantillon de sang est choisi dans le groupe constitué des globules rouges, du sang complet, des globules rouges dilués avec une solution physiologique ou une solution tampon phosphate isotonique, et du sang complet dilué avec une solution physiologique ou une solution tampon phosphate isotonique.

5. Procédé selon la revendication 3, dans lequel l'échantillon de sang est choisi dans le groupe constitué d'un échantillon de sang qui est partiellement hémolysé et d'un échantillon de sang qui est riche en lipides.

6. Procédé selon la revendication 3, dans lequel l'anticorps est fixé sur la bande de test par adsorption physique ou réticulation chimique.

7. Procédé selon la revendication 3, dans lequel la présence d'agglutination sur le site de chargement en tant que site réactionnel peut être déterminée visuellement ou à l'aide d'un dispositif recueillant un signal optique ou un signal électrique.
